# FASCICULE DE BREVET EUROPEEN

(11) **EP 0 766 692 B1**
(45) Date de publication et mention de la délivrance du brevet: **21.10.1998**
(21) Numéro de dépôt: 95923407.1
(22) Date de dépôt: 16.06.1995
(51) Int. Cl.: C07H 19/20, A61K 31/70

(54) **DERIVES DE L'ACIDE GUANYLIQUE ET APPLICATIONS COMME MEDICAMENTS**
GUANYLSÄURE DERIVATE UND IHRE VERWENDUNG ALS ARZNEIMITTEL
GUANYLIC ACID DERIVATIVES AND THEIR USE AS DRUGS

(30) Priorité: 17.06.1994 FR 9407490
(43) Date de publication de la demande: 09.04.1997
(73) Titulaire: Sté de recherche auvergnate pour l'innovation en oligothérapie inoligo, 63400 Chamalières (FR)
(72) Inventeur: NGUYEN, Dat, Xuong, F-92160 Antony (FR); RAPIN, Jean, Robert, F-75015 Paris (FR); LAMBROPOULOS, Patrick, F-13008 Marseille (FR); DAVER, Jean, F-81710 Saix (FR)
(74) Mandataire: Phélip, Bruno
(86) Numéro de dépôt international: FR9500806
(87) Numéro de publication internationale: WO9535305

(56) Documents cités:
- 'THE MERCK INDEX - eleventh edition' , MERCK AND CO., INC. , RAHWAY, N.J., U.S.A. cité dans la demande Compound 4484 - le sel monohydrate disodium d'acide 5'-guanylique et le sel octahydrate de barium d'acide 5'-guanylique

## Description

L'invention a pour objet des dérivés de l'acide guanylique et les applications de ces substances comme médicaments.

La molécule d'acide 5'-guanylique ainsi que les procédés d'obtention de cette molécule sont déjà décrits dans la littérature. On trouve notamment la référence de ce produit dans l'index Merck 11ème édition (Référence n°4484). On sait que cette molécule sous forme de sel de sodium peut être utilisée comme aromate.

De plus, cette molécule possède un rôle physiologique important. L'acide guanylique est en effet un précurseur du GMP cyclique dont le rôle dans la microcirculation est connu.

Il existe un équilibre physiologique d'une part entre adénosine et guanosine et d'autre part entre deux cations le calcium et le magnésium. L'excès de calcium intracellulaire et d'adénosine observé chez les personnes âgées se traduit par un ralentissement de l'activité intellectuelle et une asthénie. Le magnésium et la guanosine auraient l'effet inverse.

L'activité thérapeutique des dérivés de l'acide guanylique n'a néanmoins jamais été décrite, à la connaissance de la demanderesse.

Il existe pourtant un besoin important et grandissant pour trouver un médicament efficace et sans effets secondaires pour traiter les troubles biologiques liés au vieillissement.

La demanderesse s'est attaché à rechercher des molécules répondant à ce besoin. Elle a montré ainsi que des dérivés de l'acide guanylique permettaient, entre autres activités, de traiter ces troubles et étaient dépourvus de toxicité.

La présente invention a pour objet des dérivés de l'acide guanylique répondant à l'une des formules générales suivantes (I) et (II) dans lesquelles Me est un métal oligoélément impliqué dans l'activité enzymatique dans des processus physiologiques, tel que le calcium (Ca), le magnésium (Mg), le cuivre (Cu), le cobalt (Co), le nickel (Ni), le zinc (Zn), le fer (Fe), le sélénium (Se), le lithium (Li), le manganèse (Mn) ou tout autre cation, mono ou divalent, chélaté par l'acide guanylique:

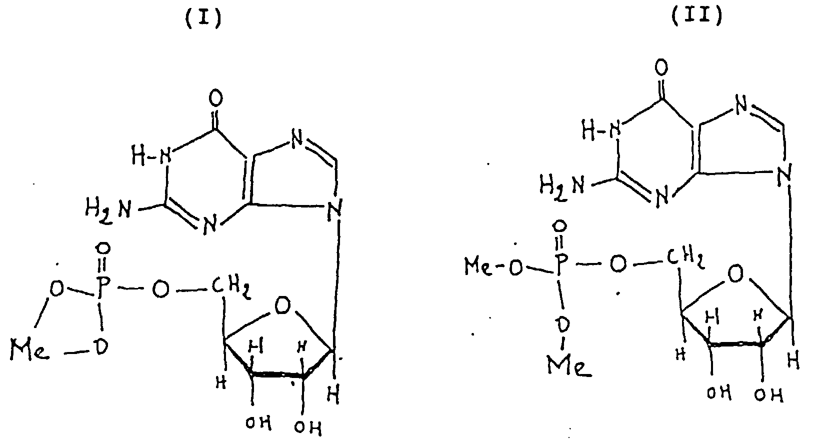

On notera que dans la formule II les deux métaux substituant le dérivé peuvent être différents.

Ces dérivés se présentent avantageusement sous forme de poudre microcristalline insoluble dans l'eau. Ils peuvent être utilisés seuls ou associés dans des formulations ou compositions acceptables pour un usage thérapeutique.

Préférentiellement de telles compositions sont gastrorésistantes, afin d'éviter l'action de l'acidité chlorhydrique de l'estomac sur les dérivés selon l'invention. De telles compositions peuvent être celles répondant aux normes de la Pharmacopée Française.

Les dérivés peuvent se présenter sous forme de mélanges racémiques ou sous forme de stéréoisomères.

La présente invention concerne aussi les formes hydratées des dérivés décrits ci-dessus en particulier celles dans lesquelles 6 à 8 molécules d'eau sont compléxées.

De manière générale, et en particulier à pH neutre, les dérivés selon l'invention sont sous forme de complexes non-ionisés. A pH acide de tels complexes sont susceptibles de se dissocier avec formation de dérivés ionisés.

Les dérivés de la présente invention peuvent être préparés en solubilisant l'acide guanylique dans un solvant organique non polaire et en ajoutant le cation sous forme de chlorure. La réaction est stoechiométrique et le rendement voisin de 100 % . Un changement de couleur montre la formation des dérivés. Après élimination des solvants, les poudres microcristallines sont lavées à l'eau pour éliminer les traces de cation qui n'ont pas réagi. Une fois séchée, la poudre se présente sous une forme microcristalline insoluble dans l'eau et les solvants polaires. Le point de fusion pour tous les composés est de l'ordre de 300°C. Le spectre RMN, l'analyse centésimale et la présence d'une seule tache par chromatographie sur couche mince confirment la structure et la pureté des dérivés obtenus. Une analyse spectrale aux rayons X montre que ces dérivés sont des chélates.

L'invention concerne également des compositions pharmaceutiques comprenant au moins un dérivé tel que décrit ci-dessus, en association avec un ou plusieurs véhicules diluants, excipients ou adjuvants compatibles et pharmaceutiquement acceptables.

De préférence, les compositions pharmaceutiques selon l'invention se présentent sous une forme appropriée pour l'administration par voie orale, parentérale ou intraveineuse.

Avantageusement, les compositions pharmaceutiques selon l'invention contiennent une quantité de dérivé tel que décrit ci-dessus, adaptée à une posologie quotidienne chez l'homme comprise entre environ 0,2 g et environ 3 g en une ou plusieurs prises.

L'invention a plus particulièrement pour objet une composition ou un médicament contenant un dérivé tel que décrit ci-dessus, et l'utilisation d'un tel dérivé, pour l'obtention de médicaments destinés au traitement:
- de l'asthénie cérébrale, par stimulation des fonctions cogénitives comme la mémoire,
- des psychasthénies, des dépressions sous leurs formes graves et légères, de l'anxiété,
- des carences en cations, tels que ceux complexés dans les composés selon la présente invention.
- des maladies vasculaires de la macro- ou de la micro circulation, telles que la vasodilatation.

Les dérivés objets de la présente invention montrent en particulier un effet vasodilatateur anti-radical et cicatrisant.

Par rapport aux molécules déjà utilisées dans le traitement des maladies et affections mentionnées ci-dessus, les dérivés selon la présente invention ont de nombreux avantages. Notamment ils sont dépourvus de toxicité et pénètrent facilement dans les cellules. De plus ils facilitent l'entrée des cations dans ces cellules par l'intermédiaire des canaux spécifiques.

L'invention est illustrée sans pour autant être limitée par les exemples qui suivent :

### EXEMPLE 1:

### Préparation du 5'-guanylate ou 5'-guanylicate de calcium.

Formule brute : C₁₀H₁₂N₅O₈PCa (et n H₂O)
Formule développée : 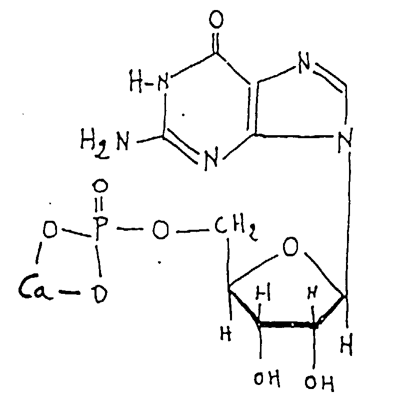
Poids moléculaire : 401 (anhydre)

| Composition centésimale | |
|---|---|
| C% | 29,95 |
| H% | 3,02 |
| N% | 17,47 |
| Ca% | 10,04 |

### Mode opératoire :

Dissoudre (en tiédissant légèrement) 0,002 mole de 0,85g de 5'-guanylate (ou 5'-guanylicate) disodique (commercialisé par Fluka sous la référence 51090) dans 10 ml d'eau distillée. Dissoudre à part 0,0025 mole de chlorure de calcium, titrant 95% ou 0,25 g dans 10 ml d'eau. Sous forte agitation, ajouter la solution calcique à la solution sodique. Instantanément un précipité incolore se forme - continuer l'agitation.

Après essorage, on lave le précipité avec de l'eau distillée pour éliminer le chlorure de sodium résiduel.

Une recristallisation est réalisée dans l'éthanol absolu (ne dissolvant pas le chlorure de sodium résiduel).

### EXEMPLE 2: Préparation du 5'-guanylate ou 5'-guanylicate de magnésium.

Formule brute: C₁₀H₁₂N₅O₈P.Mg (avec n H₂O)
Poids moléculaire : 385 (anhydre)

| Composition centésimale : | |
|---|---|
| C% | 31,19 |
| H% | 3,14 |
| N% | 18,19 |
| Mg% | 6,32 |

### EXEMPLE 3:

### Préparation de 5'-guanylate ou 5'-guanylicate de Manganèse

Formule brute : C₁₀H₁₂N₅O₈P.Mn (avec n H₂O)
Poids moléculaire : 415,93 ou 416 (anhydre)

| Composition centésimale | |
|---|---|
| C% | 28,85 |
| H% | 2,91 |
| N% | 16,84 |
| Mn% | 13,24 |

### EXEMPLE 4 :

### Préparation de 5'-guanylate ou 5'-guanylicate de Cuivre (cuivreux)

Formule brute : C₁₀H₁₂N₅O₈P.Cu (avec n H₂O)
Poids moléculaire : 424,50 anhydre

| Composition centésimale: | |
|---|---|
| C% | 28,29 |
| H% | 2,85 |
| N% | 16,50 |
| Cu% | 14,97 |

### EXEMPLE 5 :

### Préparation de 5'-guanylate ou 5'-guanylicate de fer (ferreux)

Formule brute : C₁₀H₁₂N₅0₈P.Fe ( avec n-H_{2O})
Poids moléculaire : 416,85 (anhydre)

| Composition centésimale : | |
|---|---|
| C % | 28,82 |
| H % | 2,90 |
| N % | 16,80 |
| Fe % | 13,48 |

### EXEMPLE 6:

### Préparation de 5'-guanylate ou 5'-guanylicate de lithium

Formule brute : C₁₀H₁₂N₅O₈F.Li₂ ( n H₂O)
Poids moléculaire : 375 (anhydre)
Formule développée:

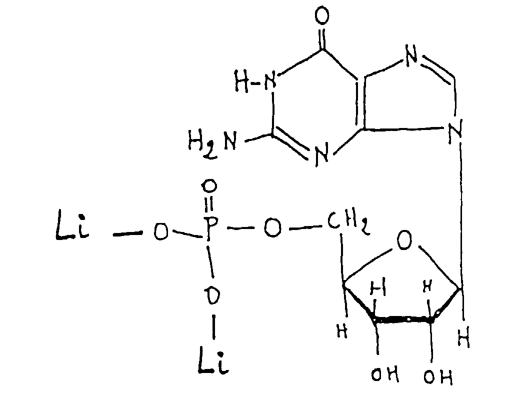

| Composition centésimale: | |
|---|---|
| C % | 32,03 |
| H % | 3,23 |
| N % | 18,68 |
| Li % | 3,70 |

### EXEMPLE 7 :

### Activité du dérivé magnésien de l'acide guanylique.

Pour montrer l'intérêt de ce produit, synthétisé dans l'exemple 2, différentes études ont été menées chez l'animal et chez l'homme.

### 1) Etudes chez l'animal.

Du point de vue toxicité aiguë, la DL 50 par voie orale chez la souris et le rat est trouvée supérieure 5g/kg. Cette toxicité est par conséquent très faible. En administration répétée pendant quinze jours chez le rat à raison de lg/kg/j aucune modification des paramètres comportementaux et biologiques n'a été observée.

Du point de vue pharmacologique, l'activité antiasthénique a été prouvée chez des rats maintenus en hypoxie normobare comme décrit par Prioux-Guyonneau et al. (J. Physiol, 1976, 72, 579-587). Cette hypoxie entraîne une diminution de la motilité et de l'activité exploratoire qui est rétablie par une seule administration une heure avant le test de 0,1 g/kg du dérivé étudié.

Une carence partielle en magnésium (40 ppm/j) mise en oeuvre comme décrit par Durlach et al. (In Magnesium Deficiency: Physiopathology and Treatment Implications, Edit. Halpern, Durlach et Kargeras 1984) provoque des désordres comportementaux, en particulier une diminution des capacités d'apprentissage observée lors d'un comportement d'évitement au son (Pole Climbing Test). Un traitement de 15 jours par voie orale avec le dérivé à la dose de 100 mg/kg/j supprime les effets de la carence sur les désordres comportementaux.

### 2. Etudes sur l'homme

Une étude clinique réalisée chez cinq patients âgés de plus de 70 ans et se plaignant de troubles de mémoire associés à une grande fatigabilité intellectuelle avec une dose quotidienne de 300 mg du dérivé présenté sous forme de comprimés pelliculés gastrorésistants, a montré une amélioration des divers symptômes et chez ces patients une reprise d'une activité cognitive normale.

Aucun effet secondaire n'a été observé chez les patients.

## Revendications

1. Dérivés de l'acide guanylique répondant à l'une des formules générales (I) et (II) suivantes: 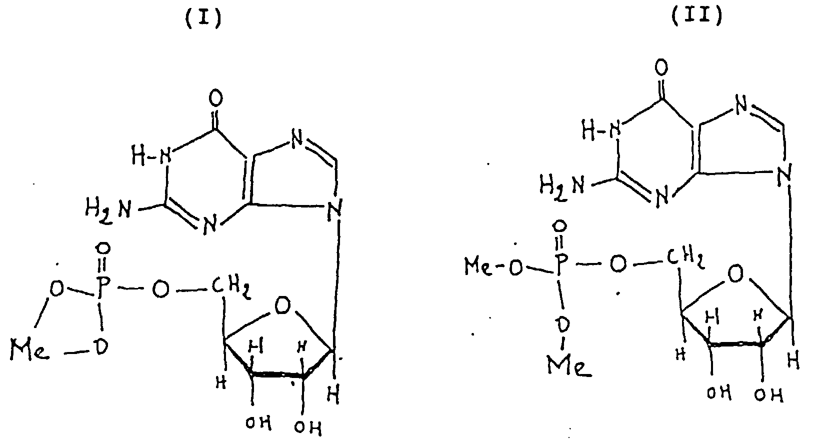 dans laquelle Me est un métal oligoélément impliqué dans l'activité enzymatique dans les processus physiologiques.

2. Dérivés selon la revendication 1, caractérisés en ce que Me est un métal pouvant former un cation monovalent ou divalent.

3. Dérivés selon l'une des revendications 1 et 2, caractérisés en ce que Me est Mg, Cu, Ca, Co, Zn, Ni, Se, Mn, Li ou Fe.

4. Dérivés selon l'une des revendications 1 à 3, caractérisés en ce qu'ils se présentent sous forme de mélanges racémiques ou sous forme de stéréo-isomères.

5. Médicament contenant au moins un des composés selon l'une des revendications 1 à 4.

6. Médicament selon la revendication 5 pour le traitement de la psychasthénie sous toutes ses formes et de la dépression sous ses formes graves et légères.

7. Médicament selon l'une des revendications 5 et 6, caractérisé en ce qu'il comprend une quantité de dérivé actif adaptée à une posologie quotidienne chez l'homme comprise entre 0,2 et 3 g.

8. Composition pharmaceutique, contenant une quantité efficace d'au moins un dérivé selon l'une quelconque des revendications 1 à 4, en association avec un ou plusieurs diluants, excipients ou adjuvants compatibles et pharmaceutiquement acceptables.

9. Composition pharmaceutique selon la revendication 8, présentée en vue de l'administration orale, parentérale ou intraveineuse.

10. Utilisation d'un dérivé selon l'une des revendications 1 à 4 pour l'obtention d'un médicament pour le traitement des psychasthénies, des dépressions, de l'anxiété, de l'asthénie cérébrale, des maladies vasculaires et des carences en cations.

## Patentansprüche

1. Guanylsäure-Derivate gemäß einer der folgenden allgemeinen Formeln (I) und (II): 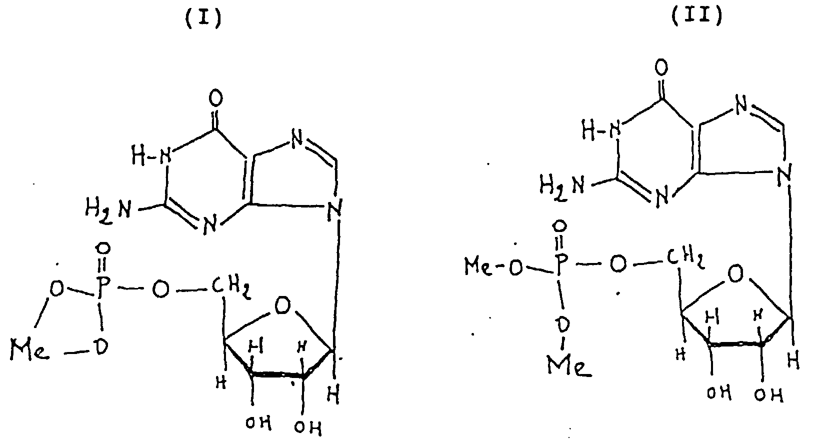 worin Me ein Spurenelement-Metall ist, das bei physiologischen Vorgängen in die enzymatische Aktivität einbezogen ist.

2. Derivate nach Anspruch 1, dadurch gekennzeichnet, daß Me ein Metall ist, das ein einwertiges oder zweiwertiges Kation bilden kann.

3. Derivate nach einem der Ansprüche 1 und 2, dadurch gekennzeichnet, daß Me Mg, Cu, Ca, Co, Zn, Ni, Se, Mn, Li oder Fe ist.

4. Derivate nach einem der Ansprüche 1 bis 3, dadurch gekennzeichnet, daß sie in Form von racemischen Mischungen oder in Form von Stereoisomeren vorliegen.

5. Medikament, enthaltend mindestens eine der Verbindungen nach einem der Ansprüche 1 bis 4.

6. Medikament nach Anspruch 5 zur Behandlung der Psychastenie in allen ihren Formen und der Depression in ihren schweren und leichten Formen.

7. Medikament nach einem der Ansprüche 5 und 6, dadurch gekennzeichnet, daß es eine Menge eines aktiven Derivats enthält, die an eine tägliche Dosierung angepaßt ist, die beim Menschen zwischen 0,2 und 3 g liegt.

8. Pharmazeutische Zusammensetzung, enthaltend eine wirksame Menge mindestens eines Derivats nach irgendeinem der Ansprüche 1 bis 4 in Verbindung mit einem oder mehreren verträglichen und pharmazeutisch annehmbaren Verdünnungsmitteln, Streckmitteln oder Adjuvantien.

9. Pharmazeutische Zusammensetzung nach Anspruch 8 für die orale, parenterale oder intravenöse Verabreichung.

10. Verwendung eines Derivats nach einem der Ansprüche 1 bis 4 zur Herstellung eines Medikaments zur Behandlung von Psychastenien, Depressionen, Angst, zerebraler Asthenie, Gefäßkrankheiten und Kationenmangel.

## Claims

1. Guanylic acid derivatives according to either of the following general formulae (I) and (II) : 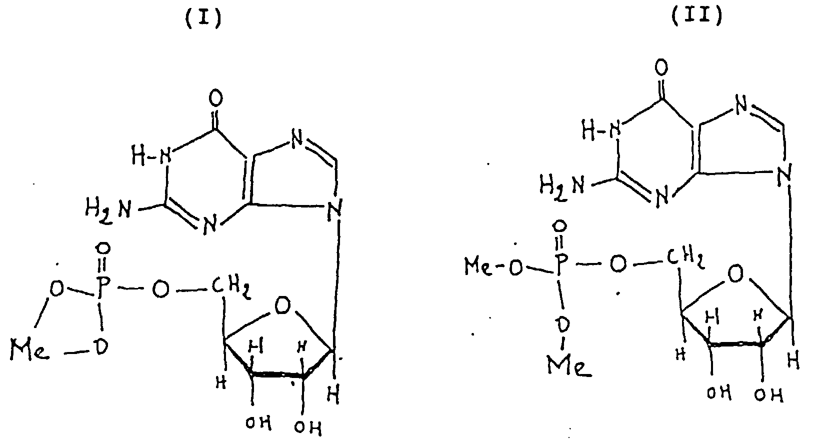 in which Me is a metal trace element involved in the enzymatic activity of physiological processes.

2. Derivatives in accordance with claim 1, characterized in that Me is a metal able to form a monovalent or divalent cation.

3. Derivatives in accordance with any of claims 1 and 2, characterized in that Me is Mg, Cu, Ca, Co, Zn, Ni, Se, Mn, Li or Fe.

4. Derivatives in accordance with any of claims 1 to 3, characterized in that they are in the form of racemic mixtures or in the form of stereoisomers.

5. Drug containing at least one of the compounds in accordance with any of claims 1 to 4.

6. Drug in accordance with claim 5 for the treatment of psychasthenia in all its forms and depression in its serious and mild forms.

7. Drug in accordance with any of claims 5 and 6, characterized in that it comprises a quantity of active derivative that is adapted to a daily dosage in man of between 0.2 and 3 g.

8. Pharmaceutical composition containing an effective quantity of at least one derivative of any of claims 1 to 4, in association with one or more diluents, excipients or additives that are compatible and pharmaceutically acceptable.

9. Pharmaceutical composition in accordance with claim 8 prepared for oral, parenteral or intravenous administration.

10. Use of a derivative of any of claims 1 to 4 to obtain a drug for the treatment of psychasthenia, depression, anxiety, cerebral asthenia, vascular illnesses and cation deficiencies.
